# EUROPEAN PATENT APPLICATION

(11) **EP 4 725 526 A1**
(43) Date of publication of application: **15.04.2026**
(21) Application number: 24205428.6
(22) Date of filing: 08.10.2024
(51) Int. Cl.: A61M 5/315, A61M 5/20, A61M 5/24

(54) **SETTING-CORRECTING MECHANISM FOR A PEN INJECTOR AND PEN INJECTOR COMPRISING SUCH MECHANISM**

(71) Applicant: Nemera Szczecin spólka z ograniczona odpowiedzialnoscia, 70-856 Szczecin (PL); NEMERA INSIGHT CHICAGO LLC, Chicago, Illinois 60640 (US)
(72) Inventor: Lundeen, John, Chicago (US); Wilczek, Mateusz, Szczecin (PL)
(74) Representative: Karcz, Katarzyna

(57) **Abstract**

A dose setting-correcting mechanism for a pen injector comprising a rotatable coupling sleeve having a proximal end (1a, 101a) and a distal end (1b, 101b), a rotatable ratchet sleeve (2, 102) surrounding the coupling sleeve (1, 101) and splined therewith axially, a reset nut (3, 103), a setting ring (6, 106), a crown gear (4, 104), said elements arranged around longitudinal axis A. For setting a dose rotation of the coupling sleeve (1, 101) in a first direction causes rotation of the ratchet sleeve (2, 102). For dose correcting the coupling sleeve (1, 101) is firstly rotated with respect to the ratchet sleeve (2, 102) and the reset nut (3, 103) is rotated along with the coupling sleeve (1, 101), wherein rotation of the reset nut (3, 103) causes sliding movement between the teeth of the reset nut and the teeth of the crown, and in result the axial movement of the crown gear (4, 104) and decoupling the crown gear (4, 104) from the setting ring (6, 106) so that the setting ring (6, 106) rotates to correct the dose. A pen injector comprising such mechanism.

## Description

### TECHNICAL FIELD

The invention relates to a dose setting and correcting mechanism for pen-type injection devices, also called pen injectors. Pen injectors are drug delivery devices used for subcutaneous injections of a medicament or other substance in a liquid form by a patient, nurse or other member of medical personnel. In a so-called variable dose pen injector, a volume of a liquid to be injected, corresponding to a given dose, can be selected by a user. Preferably, such volume can be increased to set the required dose and decreased to correct a dose that has been set too large before making the injection, more preferably in a broad range of volumes and with the small single increment of a dose for precise selecting the volume.

The present invention relates to an internal mechanism for pen injectors which allows both setting and correcting of a dose before injection.

### PRIOR ART

Dose setting and correcting mechanisms in pen injectors are known in the prior art. International patent application WO99/38554A1 discloses a pen injector in which a dose can be set by rotation of a dose setting button with respect to a bushing, which is rotationally fixed in a housing during dose setting. The bushing comprises a radial protrusion which clicks from one recess of the dose setting button wall to another recess during dose setting. By moving the radial protrusion by one position, the dose is increased by a single increment or decreased by the same value if the dose setting button rotates in the opposite direction. Rotation in both directions, corresponding to the dose setting and dose correcting, is performed in the same way as the coupling between the radial protrusion and the recesses is bidirectional. The system is, however, not precise enough, as it need to utilize large enough parts to avoid the risk of unintended displacement of the radial protrusion and therefore unintended change of the dose volume, because there is no other element preventing it. Also, such mechanism cannot be used in spring-driven injectors, because the protrusion has to operate in both directions and cannot hold the system against the bias of the spring. Besides that, the click sounds are the same for dose setting and dose correcting, so that the user is not able to use such audible feedback to distinguish between these two operations.

A dose setting and correcting mechanism applicable to the spring-driven injection devices is disclosed in WO2010/089418A2. The system is based on a ratchet mechanism utilizing the pawls moving between subsequent edges of a ratchet teeth ring. Due to the shape of the pawls and teeth, such movement is possible only in one direction, corresponding to the dose setting. When the pawls are engaged with the teeth of the ring, they cannot be rotated in the opposite direction, hence this coupling can be used for holding the mechanism against a bias from a tensioned spring. Dose correcting is achieved by radial displacement of the pawls causing further disengagement from the teeth. The pawls are moved in both directions by the corresponding steering recesses which are shaped so that the recesses rotate the pawls in one direction, corresponding to the dose setting, and the recesses causes radial deflection of the pawls in the opposite direction. When the pawls become disengaged from the teeth, the tensioned spring rotates the system back and the pawls engage the teeth again in the position, which is moved back in the ring by one position, so that the dose is corrected by one increment. While the ratchets providing unidirectional, precise and robust coupling are an advantageous solution in pen injectors in general, this particular setting mechanism requires also a large elastic deformation of the ratchet pawls in radial direction during dose correcting. This results in an additional burden in designing such a mechanism as it requires specific properties from the material used for manufacturing such ratchet pawls. Besides that, the steering recesses must have a very specific shape as they cooperate with the pawls in both directions in different ways. The manufacturing process of such elements is therefore complex and less cost-efficient. Even a small change in the conduct of such a process, for example a change in the injection molding parameters, may result in the elements which will not perform their function properly and have distorted flexibility and shape with a negative impact on the overall performance.

The patent application WO2017/064275A1 concerns a setting mechanism without any ratchets and based only on the cooperation of the stiff teeth sets. Also, contrary to the radial system of WO2010/089418A2, the solution of WO2017/064275A1 operates axially during both dose setting and dose correcting. The system is based on the teeth which could be moved with respect to the other teeth only in one direction due to their shape. When the first set of teeth are to be moved in the second direction for performing dose correcting, the another, third set of teeth have to be used for temporary decoupling of the cooperating teeth. Hence the teeth are used for both controlling the coupling and measuring the dose : a change of the dose by a single increment corresponds to the change of the position between the two sets of teeth by one unit. But for the small increments, which are required for many applications of the pen injectors, in particular administration of insulin or GLP-1 analogs therapies, the corresponding extent of rotation is small and therefore the subsequent teeth need to be close to each other. This in turn requires high precision during the manufacturing of the small teeth which is costly and relates with a higher risk of errors.

None of the prior art solutions provides a precise and reliable dose setting and correcting mechanism, which would also be robust, simple and cost-efficient in manufacture. There is a need to improve the known solutions by providing a dose setting and correcting mechanism which will not require too small elements for counting the increments and will not be based on the elastic deformation of the ratchet arms for the purpose of correcting a set dose. There is also a need for a system which could be used in broad class of the pen injectors, in particular which could be also used in the spring-driven devices.

### SUMMARY OF INVENTION

According with the invention a dose setting-correcting mechanism for a pen injector is provided, comprising a rotatable coupling sleeve having a proximal end and a distal end, a rotatable ratchet sleeve surrounding the coupling sleeve and splined therewith axially, a reset nut, a setting ring, a crown gear, wherein said coupling sleeve, ratchet sleeve, reset nut, setting ring and crown gear are arranged around the longitudinal axis. The dose setting-correcting mechanism is configured for setting a dose by rotation of the coupling sleeve in a first direction which causes rotation of the ratchet sleeve. The coupling sleeve is provided at its distal end with one element of a first pair comprising at least one ratchet arm and the ratchet teeth projecting transversally to the axis and the reset nut is provided with the other of the said first pair so that a unidirectional rotational coupling is provided between the coupling sleeve and the reset nut. Further the ratchet sleeve is provided at its distal end with one element of a second pair comprising at least one ratchet arm and the ratchet teeth projecting transversally to the axis and the setting ring is provided with the other of the said second pair, so that a unidirectional rotational coupling is provided between the ratchet sleeve and the setting ring. During dose setting the coupling sleeve rotates with respect to the reset nut and the ratchet sleeve rotates with respect to the setting ring, wherein the reset nut and the crown gear are coupled by the first cooperating sets of axially extending teeth so that a unidirectional rotational coupling is provided between the reset nut and the crown gear. The crown gear and the setting ring are releasably coupled by the second cooperating sets of the axially extending teeth, wherein during dose correcting the coupling sleeve is firstly rotated with respect to the ratchet sleeve and the reset nut is rotated along with the coupling sleeve, and rotation of the reset nut causes sliding movement between the teeth and in result the axial movement of the crown gear and decoupling the crown gear from the setting ring so that the setting ring rotates to correct the dose.

In this way a setting mechanism is provided, in which the increments are counted by the ratchet element, but said element changes its position with respect to the teeth ring only during dose setting and move together with said ring during dose correcting. Hence there is no need to deform the ratchet element for dose correcting and this element has therefore a simpler shape. The teeth are used for decoupling the system for the purpose of dose correcting, but there is no need to use coupling based on the small teeth to count the dose. On the contrary the dose is counted by the flexible ratchets which may be formed as larger element.

The setting ring may comprise a proximal part and distal part fixed to each other. In this way these two elements can be manufactured separately, which may be favorable for manufacturing process.

The teeth for coupling the crown gear and the reset nut may comprise inclined surfaces which are curved. The curved teeth facilitate relative sliding movement between these sets of teeth by reducing the friction.

The crown gear and the setting ring may comprise further sets of teeth for stopping rotation during dose correcting, said sets of teeth being spaced apart from each other along the longitudinal axis during dose setting and coupled with each other during dose correcting. In this way the stopping effect of these cooperating teeth is utilized to ensure that the rotation of the setting ring during dose correcting will be stopped in the correct position after each act of reducing of the set dose by one increment.

The teeth for stopping rotation may be spaced circumferentially.

The teeth for coupling the crown gear with the setting ring may be symmetrical. In this case transfer of rotation between these teeth is possible so that the teeth can be used also for driving the crown gear during injection.

Another aspect of the present invention is a pen injector comprising the dose setting-correcting mechanism as described above.

The pen injector may further comprise a housing adapted to be coupled with a holder for medicament cartridge, a driving sleeve coupled to a push button at its proximal end and an externally threaded piston rod. The driving sleeve is coupled in rotation with the crown gear and the driving sleeve is releasably coupled with the housing, so that the driving sleeve is rotationally fixed during both dose setting and dose correcting and is released for injecting the dose, wherein rotation of the driving sleeve causes distal displacement of the piston rod.

Such pen injector allows therefore to deliver a dose, preferably dose of medicament, whose volume can be set by the user before injection. The dose setting-correcting mechanism enables precise selection of this dose and the driving sleeve further transfers rotation onto the threaded piston and this rotation is accompanied by the axial displacement of the piston rod in the distal direction.

The pen injector may further comprise a drive spring for providing the force for injection. Use of the drive spring does not require providing the force by the user, hence lower force is needed for device activation.

The drive spring may be a helical torsion spring. The helical springs are cheaper and easier to mount to other elements than, for example, clock springs and use of the torsion load allows to obtain constant force of the injection over a broad range of the spring tension.

The drive spring may be coupled with the ratchet sleeve.

The driving sleeve may be releasably directly coupled to the housing.

The pen injector may comprise a proximally located button. Such location of the button facilitates grasping the device by the user and convenient use of the thumb for pressing the button.

The pen injector may be disposable when it is to be discarded, for example after the cartridge is empty or may be reusable allowing cartridge exchange. The pen injector may also use other, than the spring, means for providing force for the injection. The pen injector may be manual, in which the force is provided by the user or may utilize motor for driving the piston rod.

The pen injectors with electronic modules are also within the scope of the present inventions. The electronic modules may be used, for example, for monitoring the pen injector operation, gathering data about this operation and transferring the data to an external device. They may also provide feedback for the user.

The pen injector may further comprise last dose stop mechanism preventing setting a dose higher than the remaining amount of drug in the cartridge. In this way the user becomes aware that the amount of drug in the cartridge is not sufficient and that the injection will not deliver sufficient amount of medicament. The user is therefore warned and can either replace a cartridge or use a new pen.

The pen injector according to the invention can, for example, be used to administer a drug in a liquid form to a patient in order to treat a wide variety of pathologies such as, for example, autoimmune diseases. or chronic diseases such as diabetes or associated disorders, cancers, hormonal insufficiency, macular degeneration, inflammation, atherosclerosis, rheumatoid arthritis, coronary syndromes, thromboembolic diseases, etc.

Thus, the term "drug" used above designates any liquid formulation capable of being administered by the pen injector. The formulation can be, for example, a solution, a gel or a fine suspension containing one or more active ingredients. These active ingredients may be, in particular, peptides (for example, insulin or GLP-1, their derivatives or analogues, amylin or its derivatives or analogues, gastric inhibitory peptides or their analogues), proteins, glycoproteins or hormones (for example hormones from pituitary gland or hypothalamus, such as gonadotropin or gonadotropin releasing hormone, desmopressin, gonadorelin, triptorelin, terlipressin, leuprorelin, buserelin, goserelin, nafarelin, lutropin, menotropin, follicle - stimulating hormone and its analogues, follitropin, parathyroid hormone, teriparatide, abaloparatide or other analogues of parathyroid hormone, calcitonin, growth hormone, somatropin, etc., active ingredients derived from hormones or nucleotides (e.g. DNA, RNA, oligonucleotides), enzymes, polysaccharides (e.g. glycosaminoglycans, hyaluronic acids, heparins, low molecular weight heparins and their derivatives, or sulfated or forms poly-sulfated of these polysaccharides), vaccines, antibodies and their analogues (for example denosumab or panitumumab) or nutritional substances. The formulations may contain these active ingredients in the form of any pharmaceutically acceptable salt or solvate as well as any necessary and appropriate excipients.

### DETAILED DESCRIPTION

The terms "axial" and "axially" used throughout this text should be understood as meaning along or in parallel to the longitudinal axis of the dose setting-correcting mechanism or the pen-injector.

The terms "distal" and "proximal" are used here in relation to various elements and they should be understood as meaning respectively "the side of the element towards the injection site when injector pen is used" and "the opposite side to distal side". The directions are marked in some figures by "D" for distal and "P" for proximal.

The term "slanted tooth" as used throughout this text defines a tooth that has two converging operational faces. The converging operational faces of the slanted tooth may both be inclined with respect to the plane transversal to the longitudinal axis of the dose setting-correcting mechanism or with respect to the plane comprising said longitudinal axis, wherein the two converging operational faces can be inclined by the same angle or by different angles. Alternatively, only one operational face may be inclined, the other one being parallel to said longitudinal axis.

### SHORT DESCRIPTION OF THE FIGURES

Fig. 1a is an exploded perspective view of the dose setting-correcting mechanism according to the first embodiment of the invention;
Fig. 1b is a longitudinal sectional view of the dose setting-correcting mechanism according to the first embodiment of the invention;
Fig. 2 is a perspective view of a coupling sleeve and its elements;
Fig. 3 is a perspective view of a ratchet sleeve and its elements;
Fig. 4 shows an embodiment of a splined connection of the ratchet sleeve with the coupling sleeve;
Fig. 5 is a perspective view of a reset nut and its parts;
Fig. 6 shows in a perspective view the arrangement of the reset nut and the coupling sleeve;
Figs. 7a and 7b are the perspective views of a crown gear;
Fig. 8a is a perspective view a proximal setting ring;
Fig. 8b is a perspective view of the distal setting ring;
Figs. 9a-9h present various stages of the operation of the dose setting-correcting mechanism according to the first embodiment;
Fig. 10a is an exploded perspective view of the dose setting-correcting mechanism according to the second embodiment;
Fig. 10b is a longitudinal sectional view of the dose setting-correcting mechanism according to the second embodiment;
Figs. 11a and 11b are the perspective views of a coupling sleeve and its elements for the second embodiment;
Fig. 12 is a perspective view of a ratchet sleeve and its elements for the second embodiment;
Figs. 13a and 13b show an embodiment of a splined connection of the ratchet sleeve with the coupling sleeve for the second embodiment;
Fig. 14 is a perspective view of a reset nut and its parts for the second;
Fig. 15 shows in a perspective view the arrangement of the reset nut and the coupling sleeve for the second embodiment;
Fig. 16 is a perspective view of a crown gear for the second embodiment;
Fig. 17 is a perspective view a setting ring for the second embodiment;
Figs. 18a-18f present various stages of the operation of the dose setting-correcting mechanism according to the second embodiment
Fig. 19 is an exploded perspective view of an example of a pen injector using the dose setting-correcting mechanism according to the first embodiment of the invention
Figs. 20a-20b are the longitudinal sectional views of the exemplary application of the dose setting-correcting mechanism according to the first embodiment of the invention in a pen injector;
Fig. 21 shows the elements, by which a piston rod cooperates with a driving sleeve;
Fig. 22 shows the connection between the driving sleeve and a nut;
Fig. 23 is a perspective view of a nut and its parts;
Fig. 24 shows the connection between the driving sleeve, the crown gear, the nut and a fixing ring;
Fig. 25 shows the elements of the indication mechanism;
Fig. 26 shows the elements of the last dose stop mechanism;
Fig. 27 is an exploded perspective view of another example of a pen injector, using the dose setting-correcting mechanism according to the second embodiment of the invention
Figs. 28a-28b are the longitudinal sectional views of the exemplary application of the dose setting-correcting mechanism according to the second embodiment of the invention in a pen injector;
Fig. 29 shows the elements, by which a piston rod cooperates with a driving sleeve;
Fig. 30 shows the connection between the driving sleeve and a housing;
Fig. 31 shows the connection between the driving sleeve and the crown gear;
Fig. 32 shows the elements of the indication mechanism.

### DETAILED DESCRIPTION OF THE FIGURES

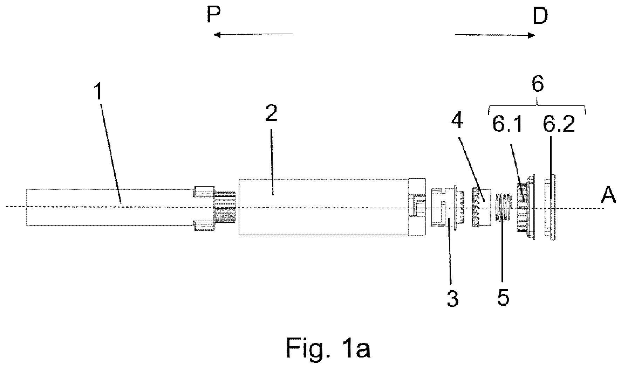

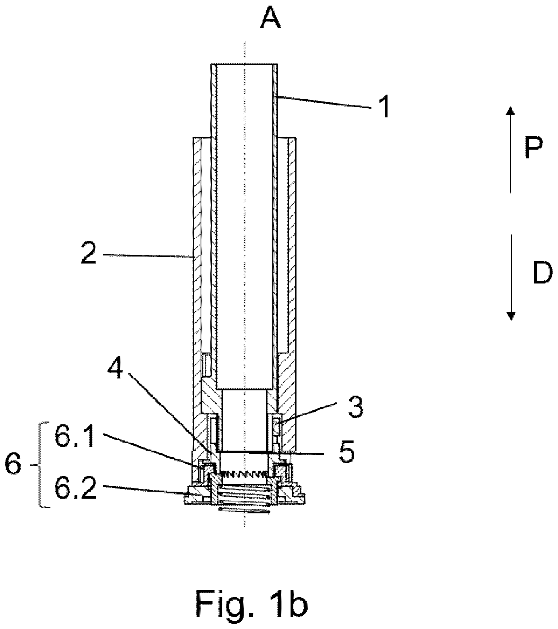

Fig. 1a shows in an exploded view the components of the dose setting-correcting mechanism according to the first embodiment of the invention, while Fig 1b shows a longitudinal cross-section of this dose setting-correcting mechanism. These components will be described in detail further with reference to the other figures.

The mechanism shown in fig. 1a comprises a rotatable coupling sleeve 1, a rotatable ratchet sleeve 2, a reset nut 3, a crown gear 4, a biasing element in form of a spring 5, and a setting ring 6 comprising in this embodiment two elements - proximal setting ring 6.1 and distal setting ring 6.2. These elements are arranged along the common longitudinal axis A.

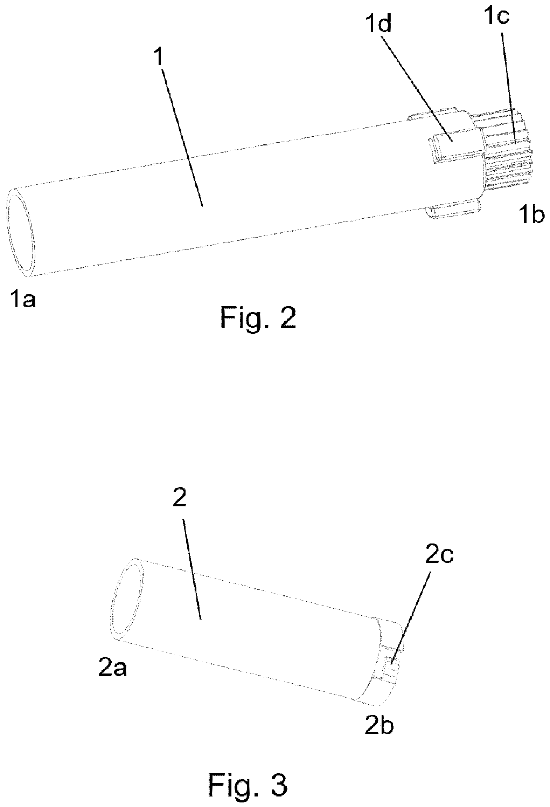

As shown in fig. 2, the coupling sleeve 1 has a proximal end 1a and a distal end 1b. The coupling sleeve 1 may be equipped with an element for rotating the coupling sleeve 1, preferably disposed close to its proximal end 1a. The distal end 1b is provided externally with circumferential ratchet teeth 1c. The circumferential ratchet teeth 1c project radially to the axis A. The coupling sleeve 1 is also provided with external protrusions 1d, located preferably near its distal end 1b.

Figs. 3 shows the ratchet sleeve 2. The ratchet sleeve 2 surrounds the coupling sleeve 1. The ratchet sleeve 2 has a proximal end 2a and distal end 2b. On its distal end 2b, the ratchet sleeve 2 is provided with at least one ratchet arm 2c. In the shown embodiment there are three ratchet arms 2c, but there may be one, two or more than three ratchet arms.

The ratchet sleeve 2 is splined with the coupling sleeve 1 in such a way that a limited relative rotation of these two elements is allowed.

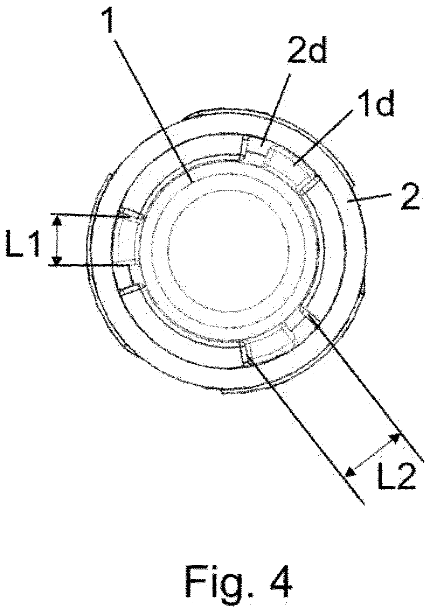

The splined connection of the ratchet sleeve 2 and the coupling sleeve 1, as realized in this embodiment, is shown in fig. 4. There are three grooves 2d formed in the internal surface of the ratchet sleeve 2 and three corresponding external projections 1d on the external surface of the coupling sleeve 1, the external projections 1d being designed to be received in the corresponding grooves 2d. The number of the grooves 2d and the number of external projections 1d can be selected by the person skilled in the art. The circumferential width L2 of the grooves 2d is larger than the circumferential width L1 of the projections 1d. The limited rotation of the coupling sleeve 1 relative to the ratchet sleeve 2 is possible as long as the projections 1d travel within the free spaces of ratchet sleeve grooves 2d, formed by the difference L2-L1.

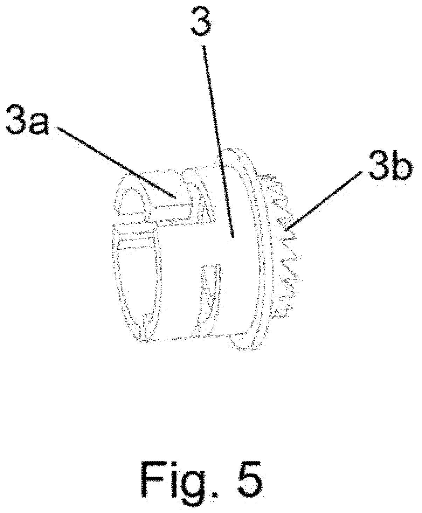

Fig. 5 shows the reset nut 3 that is provided on its proximal side with the reset nut ratchet arms 3a. In the presented embodiment, the reset nut 3 comprises three ratchet arms 3a, but the reset nut 3 can comprise at least one ratchet arm 3a. On its distal side, the reset nut 3 is provided with a reset teeth ring 3b, its teeth being slanted and projecting in the distal direction. The ratchet arms 3a of the reset nut 3 are designed for engagement with the ratchet teeth 1c of the coupling sleeve 1. Due to the inclined surfaces of the ratchet teeth 1c allowing the passage of the nut ratchet arms 3a only in one rotational direction, a unidirectional rotational engagement is formed between the coupling sleeve 1 and the reset nut 3. Alternatively, the unidirectional coupling between the reset nut 3 and the coupling sleeve 1 may be realized by means of internal ratchet teeth on the proximal side of the reset nut 3 and at least one ratchet arm at the distal end 1b of the coupling sleeve 1.

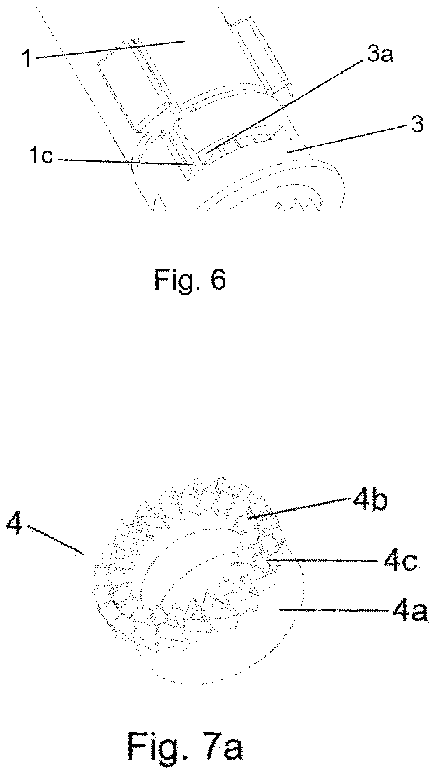

The arrangement of the reset nut 3 and the coupling sleeve 1 is best shown in fig 6.

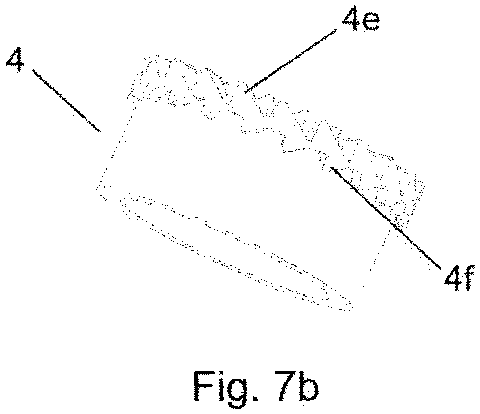

The crown gear 4 as shown in figs. 7a and 7b comprises a tubular body 4a provided at its proximal side with an internal sliding crown teeth ring 4b and an external teeth ring 4c surrounding the internal sliding crown teeth ring 4b. The teeth of internal sliding crown teeth ring 4b are slanted and project in the proximal direction.

As best shown in fig. 7b, the external teeth ring 4c comprises two sets of inclined teeth projecting in opposite directions. The first set of slanted teeth 4e projects proximally and the second set of slanted teeth 4f projects distally. The bases of the teeth of the second set of slanted teeth 4f are spaced apart from each other.

The crown gear 4 is prevented from rotation and it is axially movable.

In the presented embodiment, the teeth 4e of the first set of the external teeth ring 4c and the teeth of the internal sliding crown teeth ring 4b are slanted in opposite directions.

The teeth of the reset teeth ring 3b and the teeth of the internal sliding crown teeth ring 4b are slanted in the same direction.

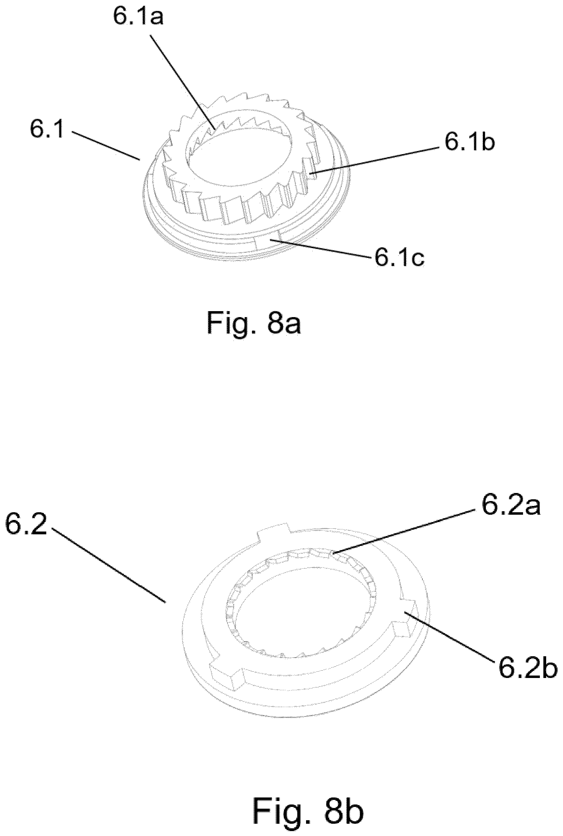

As shown in figs. 8a, the proximal setting ring 6.1 is a generally annular element provided with an internal sliding teeth ring 6.1a and an external ratchet teeth ring 6.1b. The teeth of the internal sliding teeth ring 6.1a project in the distal direction and comprise inclined surfaces. The external ratchet teeth 6.1b project transversally to the axis A and they are designed for engagement with the ratchet arms 2c of the ratchet sleeve 2. Due to the inclined surfaces of the teeth of the external ratchet teeth ring 6.1b, allowing the passage of the ratchet arms 2c only in one rotational direction, a unidirectional rotational engagement is formed between the ratchet sleeve 2 and the proximal setting ring 6.1. Alternatively, the unidirectional rotational engagement between the ratchet sleeve 2 and the proximal setting ring 6.1 may be realized by means of internal ratchet teeth on the distal side of the ratchet sleeve 2 and at least one ratchet arm provided on the proximal setting ring.

The distal setting ring 6.2 shown in fig. 8b is a generally annular element provided with an internal stopping teeth ring 6.2a, said teeth projecting in the proximal direction and comprising inclined surfaces. In the presented embodiment, the bases of the teeth of the internal stopping teeth ring 6.2a are spaced apart from each other by.

The proximal setting ring 6.1 and the distal setting ring 6.2 are fixedly engaged to each other, preferably by means of suitable projections and corresponding notches provided on the circumference of the respective elements. In the shown embodiment, the proximal setting ring 6.1 has the notches 6.1c and the distal setting ring 6.2 has the projections 6.2b.

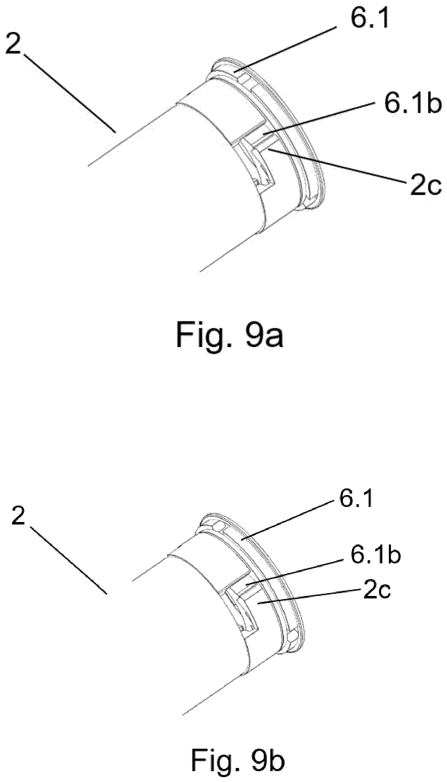

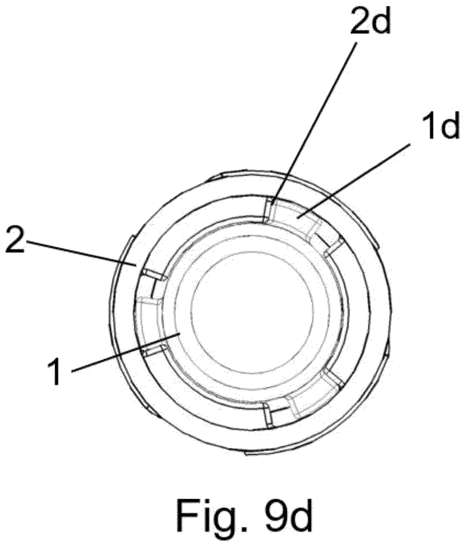

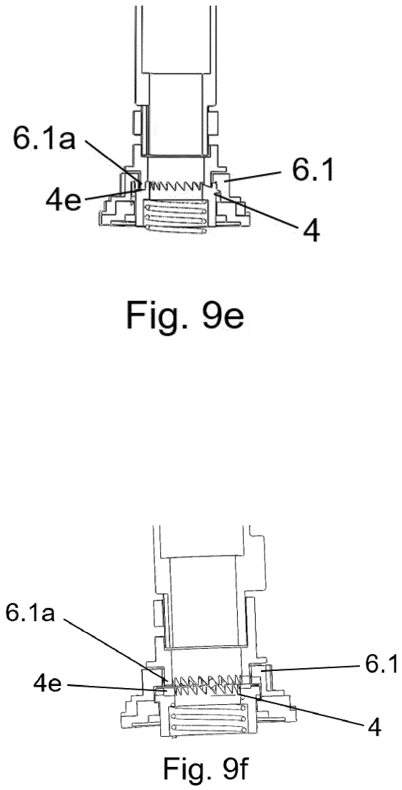

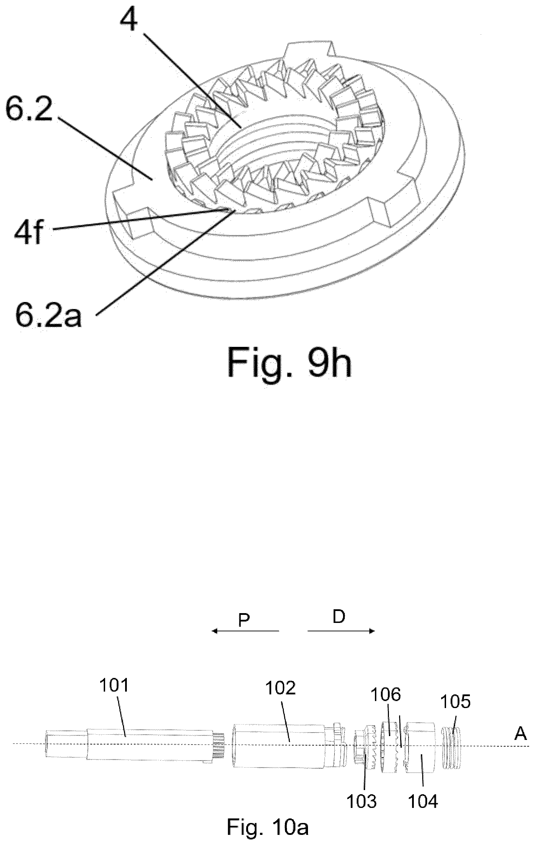

The operation of the dose setting-correcting mechanism according to the shown embodiment will now be described with reference to the figures 9a-9h.

The dose is set starting from a position of the mechanism in which the reset nut 3, the crown gear 4 and the proximal setting ring 6.1 along with the distal setting ring 6.2 are immobilized in relation to each other. In this position, the crown gear 4 is in its proximal position in which the internal sliding crown teeth ring 4b is engaged with the reset teeth ring 3b and the first set of the slanted teeth 4e is engaged with the internal sliding teeth ring 6.1a.

The dose is set by rotation of the coupling sleeve 1 in a first direction.

As explained above, the ratchet arms 3a of the reset nut 3 provide an unidirectional rotational engagement with the ratchet teeth 1c of the coupling sleeve 1. The direction of the engagement, defined by the direction of inclination of the ratchet teeth 1c, is such that when the coupling sleeve 1 is rotated in the first direction, its ratchet teeth 1c pass over the ratchet arms 3a of the reset nut 3 and the reset nut 3 does not move.

As the coupling sleeve 1 is rotated in the first direction, the ratchet sleeve 2, which is splined with the coupling sleeve 1, is also rotated and its ratchet arms 2c pass over the subsequent teeth of the external ratchet teeth ring 6.1b, so that a dose corresponding to the angle of rotation of the ratchet sleeve 2 is set. Two subsequent positions of the ratchet arms 2c are shown in Figs. 9a-9b.

As explained above (see fig. 4), the splined connection of the ratchet sleeve 2 with the coupling sleeve 1 is formed by the grooves 2d and the corresponding protrusions 1d. There is also a free space in each groove 2d. The free space could be travelled by the protrusions 1d of the coupling sleeve 1 only in the direction corresponding to dose correcting. The extent of available movement is determined by an angle a defined by the circumferential width L1 less the circumferential width L2.

The dose may be corrected by rotation of the coupling sleeve 1 in the second direction, opposite to the first direction.

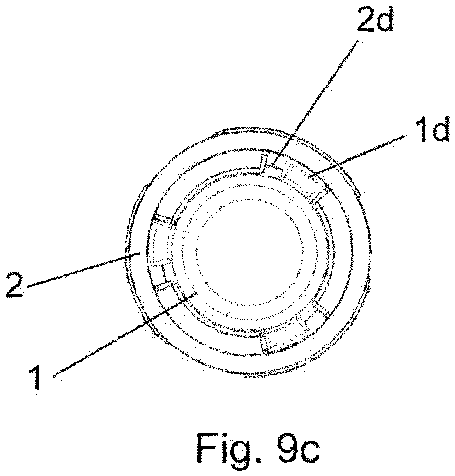

When the coupling sleeve 1 is rotated in the second direction, the protrusions 1d firstly travel the angle a, hence the ratchet sleeve 2 is not rotated and it stays immobilized by its ratchet arms 2c engaged with the external ratchet teeth ring 6.1b. This first stage of dose correcting is shown in the Figs. 9c-9d. As the ratchet arms 3a of the reset nut 3 are unidirectionally engaged with the ratchet teeth 1c of the coupling sleeve 1, the reset nut 3 is rotated in the second direction along with the coupling sleeve 1. As the reset nut 3 is rotated, the teeth of its reset teeth ring 3b slide on the teeth of the internal sliding crown teeth ring 4b, causing the movement of the crown gear 4 in the distal direction to its distal position. During this movement the spring 5 is compressed. When the crown gear 4 is in its distal position, the first set of the slanted teeth 4e are disengaged from the teeth of the coupling teeth ring 6.1a. The disengagement process is shown in Figs. 9e-9f. As a result, the proximal setting ring 6.1 is free to rotate back in the second direction together with the ratchet sleeve 2 and its ratchet arms 2c. The rotation of the ratchet arms 2c in the second direction would be prevented by the shape of the teeth of the external ratchet teeth ring 6.1b, if the coupling sleeve 1 is not first rotated to decouple the system and allow the common rotation of the proximal setting ring 6.1 and the ratchet arms 2c.

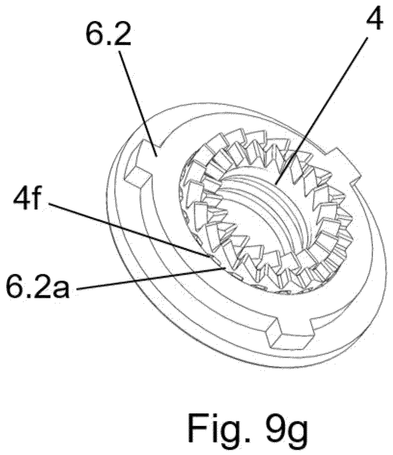

During dose correcting, the distal setting ring 6.2 rotates along with the proximal setting ring 6.1 as they are both fixedly coupled. The rotation of these elements along with the ratchet sleeve 2 is stopped by engagement of the internal stopping teeth ring 6.2a with the second set of slanted teeth 4f. This is shown in Figs. 9g-9h. After this engagement, the force generated by the spring 5 moves the crown gear 4 back in the proximal direction, and the first set of the slanted teeth 4e engage again the teeth of the coupling teeth ring 6.1a. This prevents further rotation of the proximal setting ring 6.1 and the described sequence corresponds to correcting a dose by a single increment, which could be repeated until the desired dose is reached.

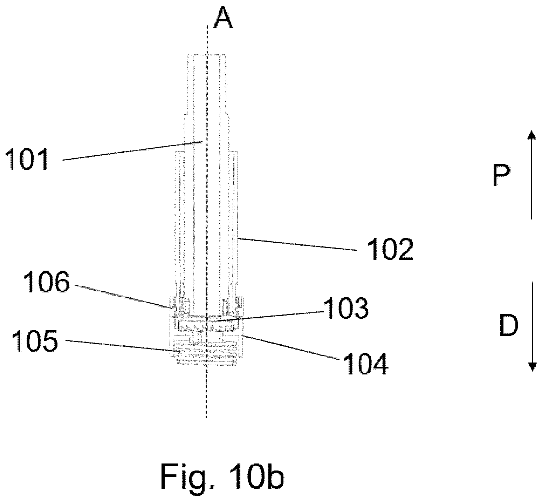

Fig. 10a shows in an exploded view the components of the dose setting-correcting mechanism according to the second embodiment of the invention, while Fig 10b shows a longitudinal cross-section of this dose setting-correcting mechanism. These components will be described in detail further with reference to the other figures.

The mechanism shown in fig. 10a comprises a rotatable coupling sleeve 101, a rotatable ratchet sleeve 102, a reset nut 103, a crown gear 104, a biasing element in form of a spring 105 and a setting ring 106. These elements are arranged along the common longitudinal axis A.

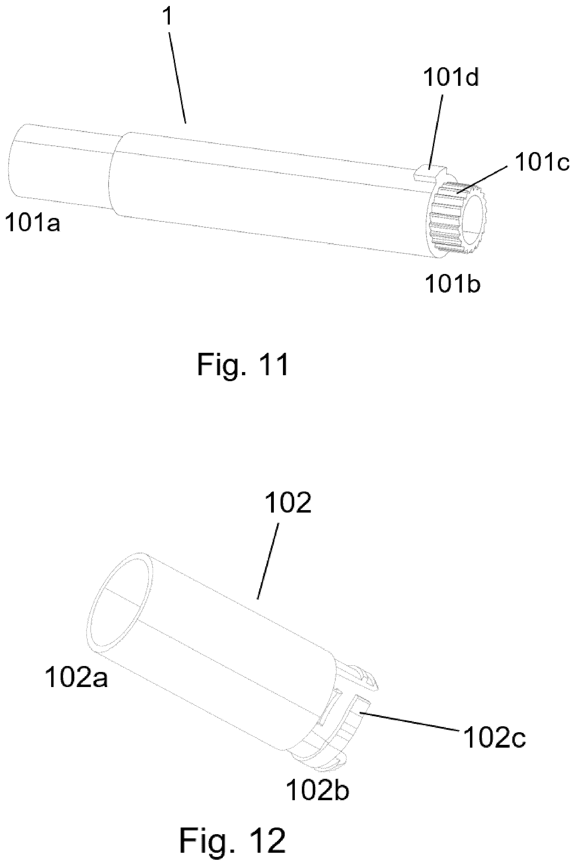

As shown in fig. 11, the coupling sleeve 101 has a proximal end 101a and a distal end 101b. The coupling sleeve 101 may be equipped with an element for rotating the coupling sleeve 101, preferably disposed close to its proximal end 101a. The distal end 101b is provided externally with circumferential ratchet teeth 101c. The circumferential ratchet teeth 101c project transversally to the axis A. The coupling sleeve 101 is also provided with external protrusion 101d, located preferably near its distal end 101b. In this embodiment, the external protrusion 101d is arc-shaped as can be seen in Fig. 11b.

Figs. 12 shows the ratchet sleeve 102. The ratchet sleeve 102 surrounds the coupling sleeve 101. The ratchet sleeve 102 has a proximal end 102a and distal end 102b. On its distal end 102b, the ratchet sleeve 102 it is provided with at least one ratchet arm 102c. In the shown embodiment there is one ratchet arm 102c, but there may be two or more ratchet arms.

The ratchet sleeve 102 is splined with the coupling sleeve 101 in such a way that a limited relative rotation of these two elements is allowed.

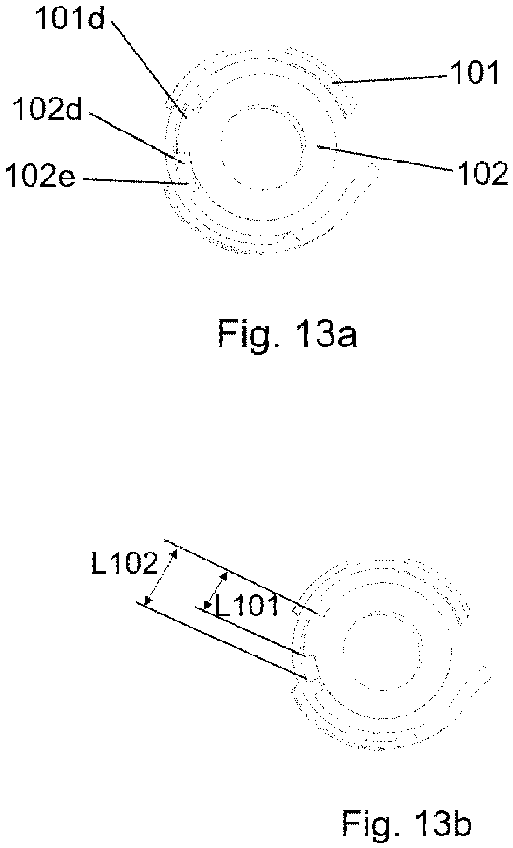

The splined connection of the ratchet sleeve 102 and the coupling sleeve 101, as realized in this embodiment, is shown in fig. 13a. There is a groove 102d formed in the internal surface of the ratchet sleeve 102 between two longitudinal projections 102e and an arc-shaped projection 101d on the external surface of the coupling sleeve 101, the projection 101d being designed to be received in the groove 102d. The number of the grooves 102d and the number projections 101d can be selected by the person skilled in the art so that there may be also two or more grooves on the internal surface of the ratchet sleeve 102 and two or more protrusions on the coupling sleeve 101. The circumferential width L102 of the groove 102d is larger than the circumferential width L101 of the projection 101d. These dimensions are shown in Fig. 13b. The limited rotation of the coupling sleeve 101 with respect to the ratchet sleeve 102 is possible as long as the projection 101d, travelling within the free spaces of ratchet sleeve grooves 102d, does not abut the protrusion 102e.

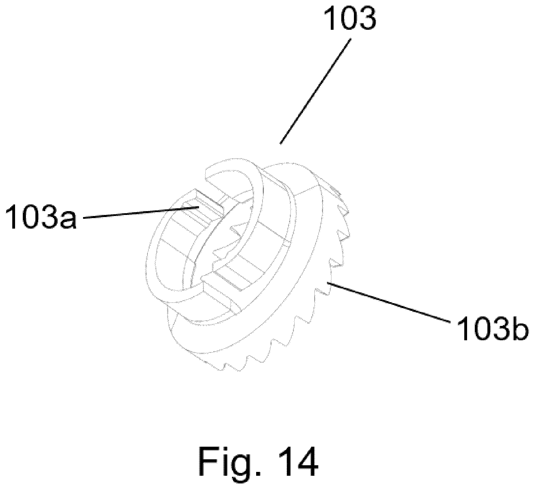

Fig. 14 shows the reset nut 103 that is provided on its proximal side with the reset nut ratchet arms 103a. In the presented embodiment, the reset nut 103 comprises two ratchet arms 103a, but the reset nut 103 can comprise, at least one ratchet arm. On its distal side, the reset nut 103 is provided with a reset teeth ring 103b, its teeth being slanted and projecting in the distal direction. The ratchet arms 103a of the reset nut 103 are designed for engagement with the ratchet teeth 101c of the coupling sleeve 101. Due to the inclined surfaces of the ratchet teeth 101c allowing the passage of the nut ratchet arms 103a only in one rotational direction, a unidirectional rotational engagement is formed between the coupling sleeve 101 and the reset nut 103. Alternatively, the unidirectional coupling between the reset nut 103 and the coupling sleeve 101 may be realized by means of internal ratchet teeth on the proximal side of the reset nut 103 and at least one ratchet arm at the distal end 101b of the coupling sleeve 1101.

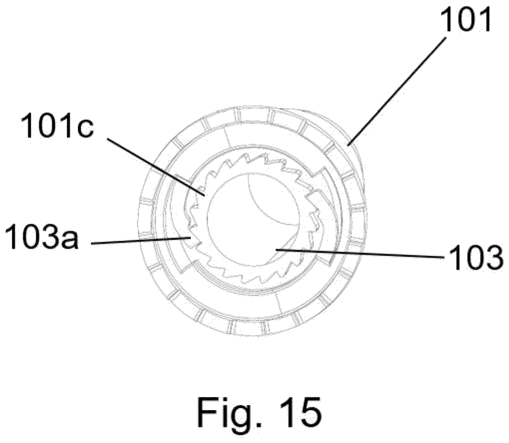

The arrangement of the reset nut 103 and the coupling sleeve 101 is best shown in fig 15.

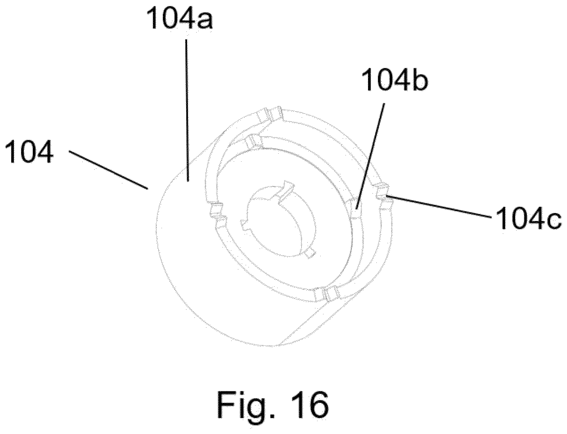

The crown gear 104 as shown in fig. 16 comprises a tubular body 104a provided at its proximal side with an internal sliding crown teeth ring 104b and an external teeth ring 104c. The external teeth ring 104c ring is disposed proximally with respect to the internal sliding crown teeth ring 104b. Both the teeth of the internal sliding crown teeth ring 104b and the external teeth ring 104c are slanted and they project in the proximal direction. In the presented embodiment there are four sets of two teeth each forming the external teeth ring 104c and base of each tooth is separated circumferentially from the base of the adjacent tooth belonging to the different set.

Alternatively the teeth of the external teeth ring 104c may be arranged in other manner, in particular they may be arranged without circumferential spacing.

The crown gear 104 is prevented from rotation and it is axially movable.

In the presented embodiment, the teeth of the external teeth ring 104c are symmetrical.

The teeth of the reset teeth ring 103b and the teeth of the internal sliding teeth ring 104b are slanted in the same direction.

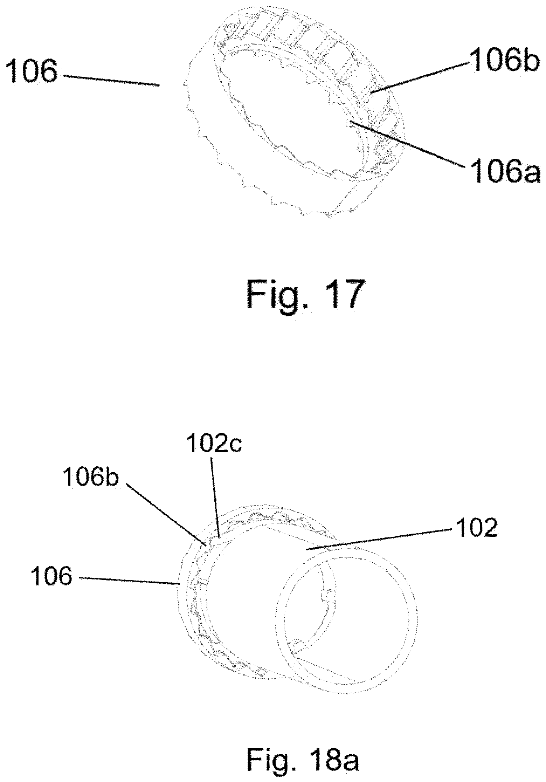

As shown in figs. 17, the setting ring 106 is a generally annular element provided with a coupling teeth ring 106a and an internal ratchet teeth ring 106b. The teeth of the internal sliding teeth ring 106a project in the distal direction and comprise inclined surfaces. The teeth of the internal ratchet teeth ring 106b project transversally to the axis and they are designed for engagement with the ratchet arm 102c of the ratchet sleeve 102. Due to the inclined surfaces of the teeth of the internal ratchet teeth ring 106b, allowing the passage of the ratchet arms 102c only in one rotational direction, a unidirectional rotational engagement is formed between the ratchet sleeve 102 and the setting ring 106. Alternatively, the unidirectional rotational engagement between the ratchet sleeve 102 and the setting ring 106 may be realized by means of internal ratchet teeth on the distal side of the ratchet sleeve 102 and at least one ratchet arm provided on the proximal side of the setting ring 106.

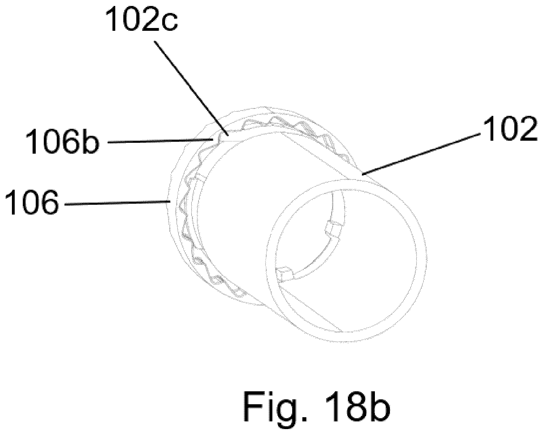

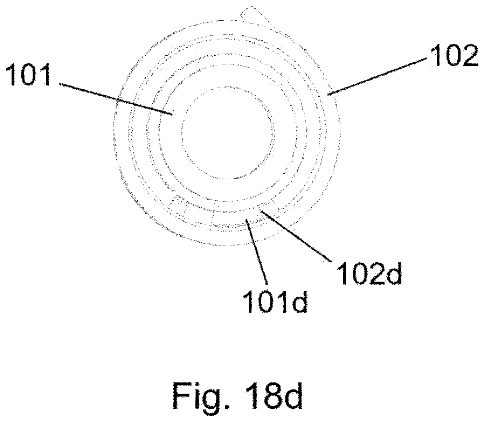

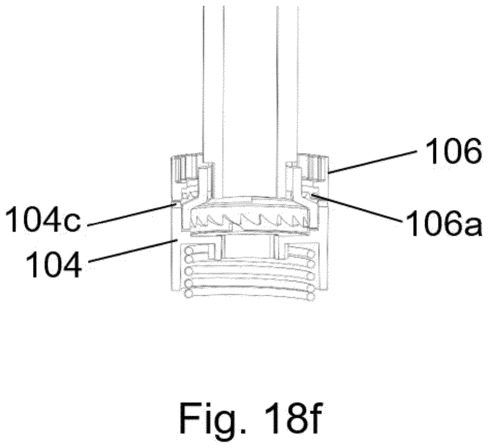

The operation of the dose setting-correcting mechanism according to the shown embodiment will now be described with reference to the figures 18a-18f.

The dose is set starting from a position of the mechanism in which the reset nut 103, the crown gear 104 and the setting ring 106 are immobilized in relation to each other. In this position, the crown gear 104 is in its proximal position in which the internal sliding crown teeth ring 104b is engaged with the reset teeth ring 103b and the external teeth ring 104c is engaged with the coupling teeth ring 106a.

The dose is set by rotation of the coupling sleeve 1 in a first direction.

As explained above, the ratchet arms 103a of the reset nut 103 provide a unidirectional rotational engagement with the ratchet teeth 101c of the coupling sleeve 101. The direction of the engagement, defined by the direction of inclination of the ratchet teeth 101c, is such that when the coupling sleeve 101 is rotated in the first direction, its ratchet teeth 101c pass over the ratchet arms 103a of the reset nut 103 and the reset nut 103 does not move.

As the coupling sleeve 101 is rotated in the first direction, the ratchet sleeve 102, which is splined with the coupling sleeve 101, is also rotated and its ratchet arm 102c passes over the subsequent teeth of the internal teeth ring 106b, so that a dose corresponding to the angle of rotation of the ratchet sleeve 102 is set. Two subsequent positions of the ratchet arms 102c are shown in Figs. 18a-18b.

As explained above (see figs. 13a-13b), the splined connection of the ratchet sleeve 102 with the coupling sleeve 101 is formed by the groove 102d and the corresponding protrusion 101d.

There is also a free space in each groove 102d. The free space could be travelled by the protrusions 101d of the coupling sleeve 101 only in the direction corresponding to dose correcting.

The dose may be corrected by rotation of the coupling sleeve 101 in the second direction, opposite to the first direction.

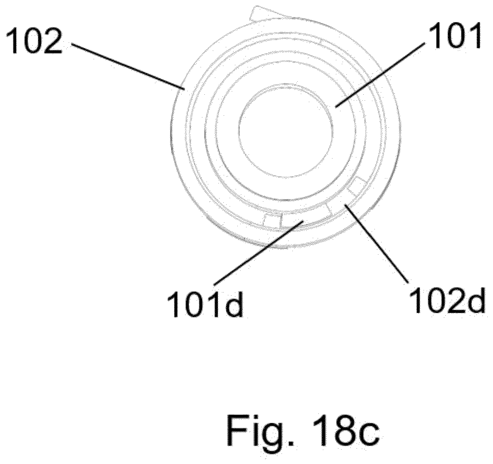

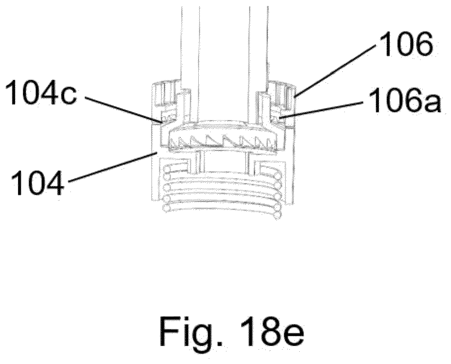

When the coupling sleeve 101 is rotated in the second direction, the protrusion 101d firstly travel the angle corresponding to the available free space in the groove 102d , hence the ratchet sleeve 102 is not rotated and it stays immobilized by its ratchet arms 102c engaged with the internal ratchet teeth ring 106b. This first stage of dose correcting is shown in the Figs. 18c-18d. As the ratchet arms 103a of the reset nut 103 are unidirectionally engaged with the ratchet teeth 101c of the coupling sleeve 101, the reset nut 103 is rotated in the second direction along with the coupling sleeve 101. As the reset nut 103 is rotated, its reset teeth ring 103b slides on the teeth of the internal sliding crown teeth ring 104b, causing the movement of the crown gear 104 in the distal direction to its distal position. During this movement the spring 105 is compressed. When the crown gear 104 is in this distal position, the teeth of the external teeth ring 104c are disengaged from the coupling teeth ring 106a. The disengagement process is shown in Figs. 18e-18f. As a result, the setting ring 106 is free to rotate back in the second direction together with the ratchet sleeve 102 and its ratchet arms 102c. Rotation of the ratchet arms 102c in the second direction would be prevented by the shape of the internal ratchet teeth ring 106b, if the coupling sleeve 101 is not first rotated to decouple the system and allow the common rotation of the proximal setting ring 106 and the ratchet arms 102c. After correcting a dose by one increment, the force from the spring 105 moves the crown gear 104 back in the proximal direction, and the external teeth ring 104c engage again the coupling teeth ring 106a. This prevents further rotation of the setting ring 106 and the described sequence corresponds to correcting a dose by a single increment, which could be repeated until the desired dose is reached.

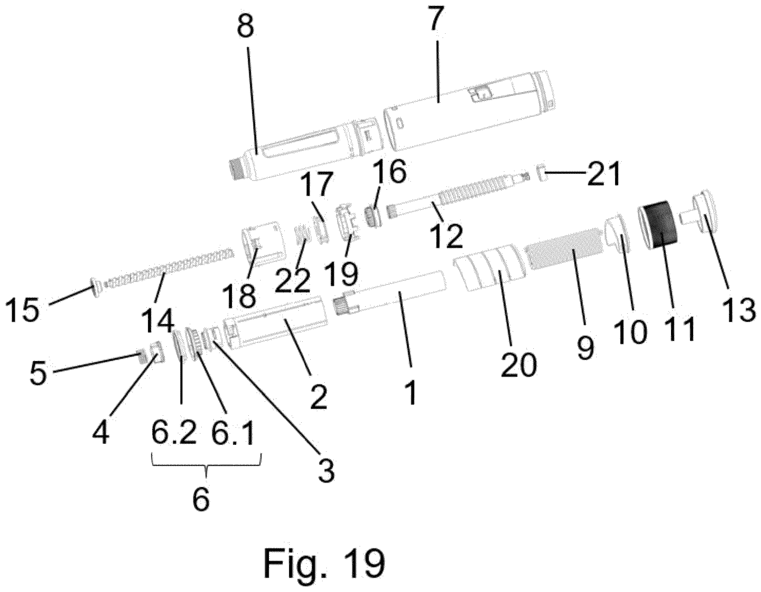

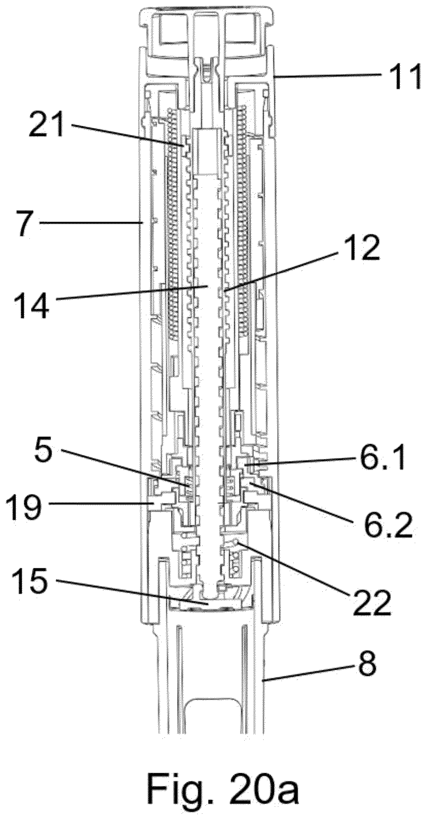

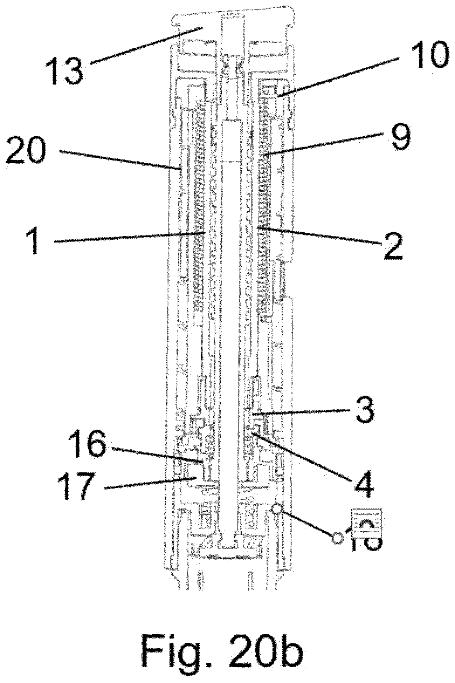

An exemplary pen injector with the dose setting mechanism according to the first embodiment of the invention is shown in an exploded view in fig. 19 and in two different longitudinal sectional views - fig. 20a and fig. 20b.

The injection pen comprises the above-described dose setting-correcting mechanism according to the first embodiment of the invention. Said mechanism is located in a housing 7 adapted to be coupled with a holder 8 adapted to receive a medicament cartridge, the housing 7 comprising an internal thread 7a. The dose setting-correcting mechanism comprises appropriate elements for engaging other parts of the pen injector as it is described below. The pen injector of this example comprises a drive spring 9, which is located between the coupling sleeve 1 and the ratchet sleeve 2. One end of the spring is preferably immobilized with respect to the housing 7, while the other end is moved during pen injector operation. In the presented example the proximal end 9a is immobilized. The proximal end 9a of the drive spring 9 is attached directly or indirectly to the housing 7. In the presented example, it is attached to an insert 10 which is fixed in the housing. The distal end 9b of the drive spring 9 is fixed to the ratchet sleeve 2. In the presented example the drive spring 9 is a helical, torsion spring, but another type of the drive spring can be used with the dose setting-correcting mechanism as well, like a clock spring or compression spring.

The proximal end 1a of the coupling sleeve 1 is connected to the element for rotating the coupling sleeve 1, which in this example is a knob 11.

The pen injector further comprises a driving sleeve 12 connected at its proximal end 12a to a push button 13.

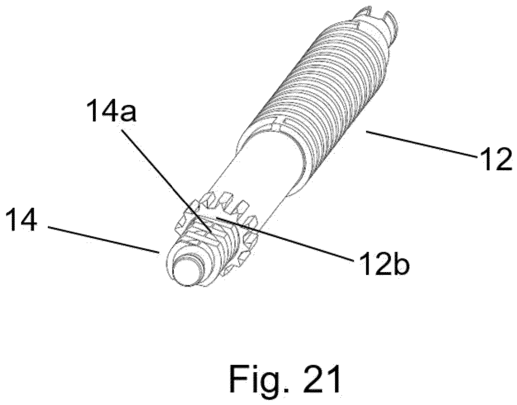

As shown in figs. 20a and 20b, the driving sleeve 12 extends through the coupling sleeve 1 of the dose setting-correcting mechanism. An threaded piston rod 14 extends through the driving sleeve 12. The piston rod 14 has a piston rod foot 15 at its distal end, the piston rod foot 15 being designed to be insertable in the cartridge. In other examples the piston rod foot may be also integral with the piston rod. The piston rod 14 is blocked in rotation within the driving sleeve 12. Preferably, in this example, the piston rod 14 comprises at least one flattened surface 14a and the driving sleeve 12 comprises at least one internal flattened surface 12b, the flattened surfaces 14a and 12b cooperating for blocking the rotation of the piston rod 14 relative to the driving sleeve 12 as it is shown in Fig. 21.

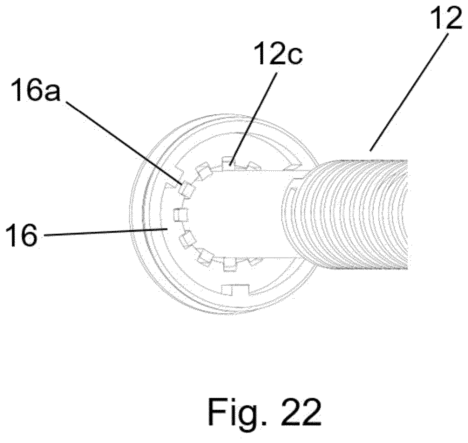

Fig. 22 shows that the distal end 12c of the driving sleeve 12 is slidingly connected with a nut 16, preferably by a multi-spline connection. The nut 16 is partly surrounded by an axially movable fixing ring 17. The nut 16 and the fixing ring 17 are detachably connected by a corresponding multi-spline.

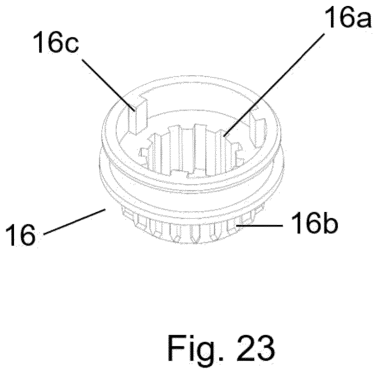

The nut 16 is shown in detail in fig. 23. It comprises the internal splines 16a for the sliding engagement with the driving sleeve 12 and an external splines 16b at its distal side for the detachable engagement with the fixing ring 17. As shown in fig. 22, the fixing ring 17 is splined to a threaded insert 18 further fixedly mounted in the housing 7, so that the fixing ring 17 is also prevented from rotation.

Further, the nut 16 comprises three longitudinal projections 16c, the number of which may be at least one. The function of the projections 16c is to be engaged with the external longitudinal grooves 4d of the crown gear 4. The engagement of the projections 16c with the grooves 4d locks the nut 16 in rotation with the crown gear 8.

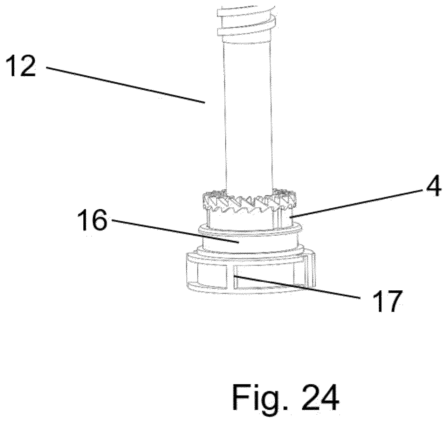

Fig. 24 shows the mutual arrangement of the driving sleeve 12, the crown gear 4, and the nut 16 when it is engaged with the fixing ring 17.

The pen injector further comprises a generally annular bearing 19 that is locked in the threaded insert 18.

The insert 18 is best shown in figs. 19 and 20a-20b. As may be seen in fig. 20b, the piston rod 14 extends through the insert 18 so that an internal thread 18a of the insert 18 cooperates with an external thread 14b of the piston rod 14.

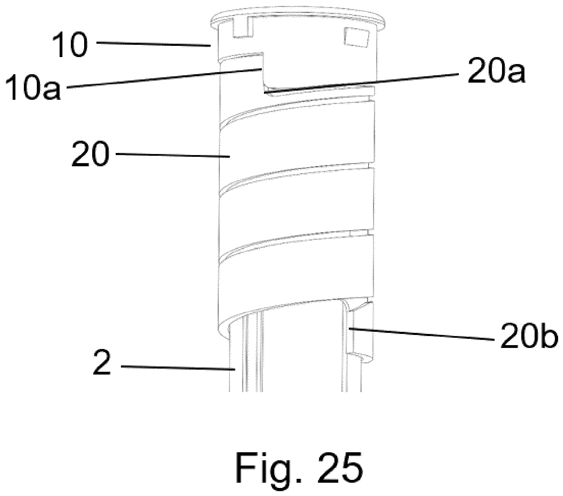

Figs. 25 show the arrangement of an indication member 20 and other parts of the injector. The indication member 20 is splined to the ratchet sleeve 2 and threadedly engaged with the housing 7 (not shown in Fig. 25). The currently set dose is visible on the indication member 20 through a window in the housing 7.

The fixed insert 10 is located within the knob 11 and it is attached inside the housing 7. As mentioned above, the drive spring 9 is attached to the fixed insert 10. The pen injector is equipped with a zero dose stop formed by the abutment of a vertical surface 20a of the indication member 20 and a corresponding vertical surface 10a of the fixed insert 10 as shown in fig. 25 showing the initial position of the indication member 20, being also the position where the indication member 20 is stopped after delivery of a dose. The indication member 20 comprises also a maximum dose stop 20b which limits the maximum dose which can be set by abutting a corresponding protrusion on the internal surface of the housing 7.

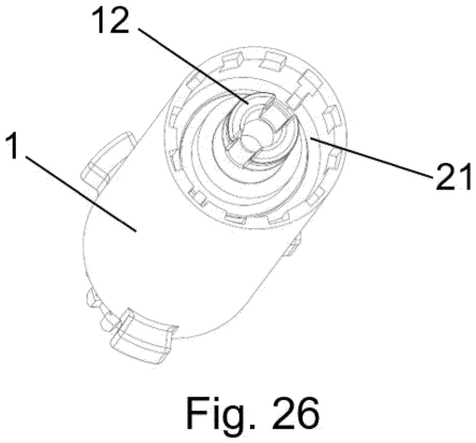

The pen injector according to the invention may be equipped with a last dose stop system, preventing setting of a dose higher than the amount of drug in the cartridge. The last dose stop system is provided by a blocking ring 21 which is shown in fig. 26 along with the elements with which it cooperates. The blocking ring 21 is rotationally coupled with the coupling sleeve 1 and it is threadedly engaged with the driving sleeve 12. During dose setting and dose correcting the coupling sleeve 1 rotates with respect to the driving element 12 so that the blocking ring 21 is rotated and moves axially in the distal direction along the thread of the driving element 12. This axial movement of the blocking ring 21 may be limited by suitable blocking means so as to limit the possibility do set a next dose. In this embodiment the blocking means are provided as end of a thread 12d on the driving sleeve 12 and the protrusion on an internal surface 21a of the blocking ring. During dose injection all the coupling sleeve 1, the driving sleeve 12 and the blocking ring 21 rotate together and their relative positions do not change. A dose is set by rotation of the knob 11 and according to the operation of the dose setting-correcting mechanism of the first embodiment as it is described above. When the set dose is to be injected, the push button 13 is pressed causing the driving sleeve 12 to be pressed as well. The distal end 12b of the driving sleeve 12 pushes the fixing ring 17 distally causing its disengagement from the nut 16. When the nut 16 is disengaged from the fixing ring 17, there is no other element to block the rotation of the nut 16. On the other hand, the nut 16 is engaged in rotation with the crown gear 4. When the dose is set and the pen injector is ready for delivery, the drive spring 9 is tensioned, and when the mechanism is not blocked any more, the spring 9 rotates the ratchet sleeve 2 and its ratchet arms 2c rotate the proximal setting ring 6.1 which in turn rotates the crown gear 4 and the nut 16. The nut 16 further rotates the driving sleeve 12 by means of their splined connection and the driving sleeve 12 rotates the piston rod 14 by means of the cooperating flattened surfaces 12b and 14a of these elements. In this way, the rotating piston rod 14 is also displaced axially in the distal direction due to its threaded coupling with the insert 18.

As may be seen in fig. 19, the pen injector according to the invention is also provided with a return spring 22 which is compressed during pressing the push button 13, because it is disposed between the fixing ring 17 and the threaded insert 18, which does not move. After dose injection, the return spring 22 pushes the fixing ring 17 back proximally to its initial position in which the fixing ring 17 engages again the nut 16 and the mechanism is ready for the next injection, if a cartridge is not empty.

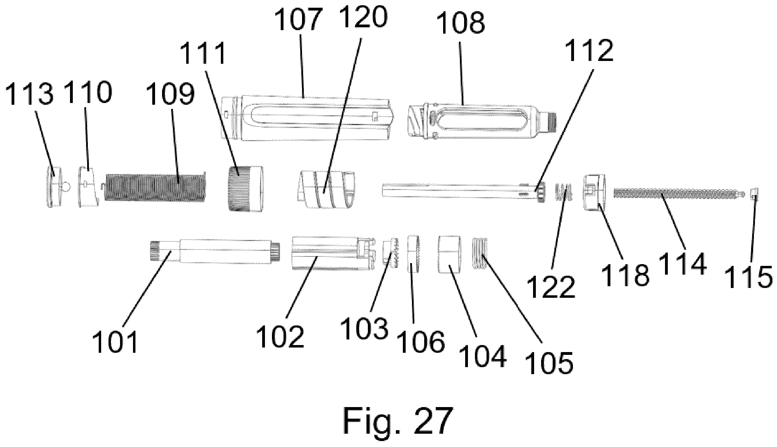

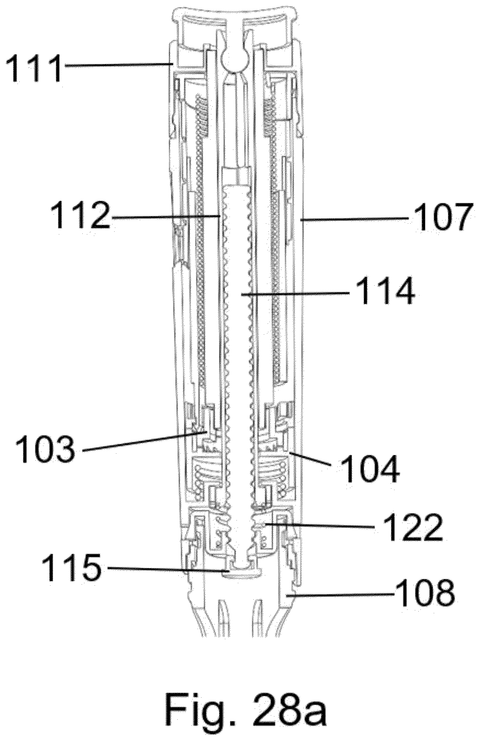

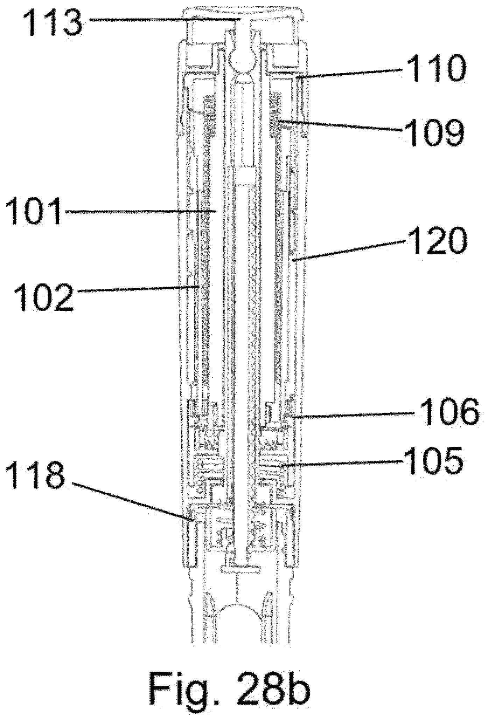

An exemplary pen injector with the dose setting mechanism according to the second embodiment of the invention is shown in an exploded view in fig. 27 and in two different longitudinal sectional views - fig. 28a and fig. 28b.

The pen injector comprises the above described dose setting-correcting mechanism according to the first embodiment of the invention. Said mechanism is located in a housing 107 adapted to be coupled with a holder 108 for a medicament cartridge, the housing 107 comprising an internal thread 107a. The dose setting-correcting mechanism elements comprises appropriate elements for engaging other parts of the pen injector as it is described below.

The pen injector comprises a drive spring 109, which is located between the coupling sleeve 101 and the ratchet sleeve 102. The proximal end 109a of the drive spring 109 is to be immobilized in relation to the dose setting-correcting mechanism. When the mechanism is located in the pen injector according to the invention, the proximal end 109a of the drive spring 109 is attached directly or indirectly to its housing. In the presented example it is attached to a fixed insert 110. The distal end 109b of the drive spring 109 is fixed to the ratchet sleeve 102. In the presented example the drive spring 109 is a helical, torsion spring, but another type of the drive spring can be used with the dose setting-correcting mechanism as well, like a clock spring or compression spring.

The proximal end 101a of the coupling sleeve 101 is connected to the element for rotating the coupling sleeve 101, which in this example is a knob 111.

The pen injector further comprises a driving sleeve 112 connected at its proximal end 112a to a push button 113.

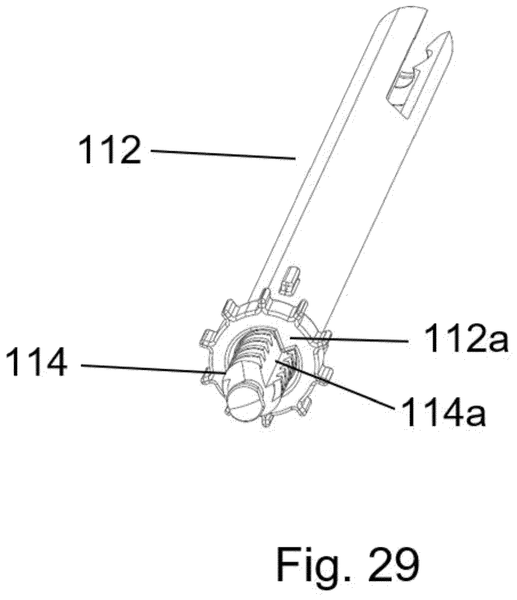

As shown in figs. 28a and 28b, the driving sleeve 112 extends through the coupling sleeve 101 of the dose setting-correcting mechanism. An threaded piston rod 114 extends through the driving sleeve 112. The piston rod 114 has a piston rod foot 115 at its distal end, the piston rod foot 115 being designed to be insertable in the cartridge. In other examples the piston rod foot may be also integral with the piston rod. The piston rod 114 is blocked in rotation within the driving sleeve 112. Preferably, in this example, the piston rod 114 comprises at least one longitudinal groove 114a and the driving sleeve 112 comprises at least one corresponding protrusion 112b, the grooves 14a and protrusions 12b cooperating for blocking the rotation of the piston rod 114 relative to the driving sleeve 112 as it is shown in Fig. 29.

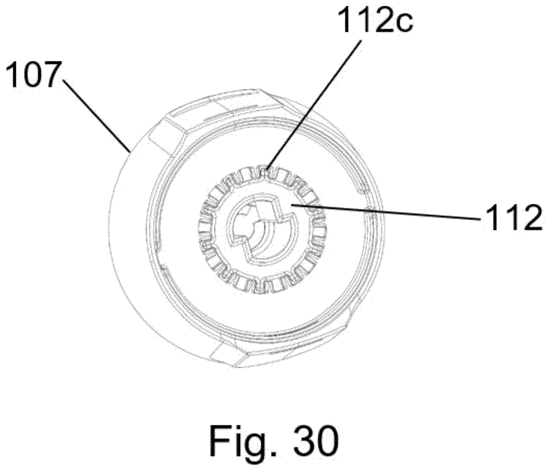

Fig. 30 shows that the distal end 112c of the driving sleeve 112 is slidingly and detachably connected with the housing 107, preferably by a multi-spline connection.

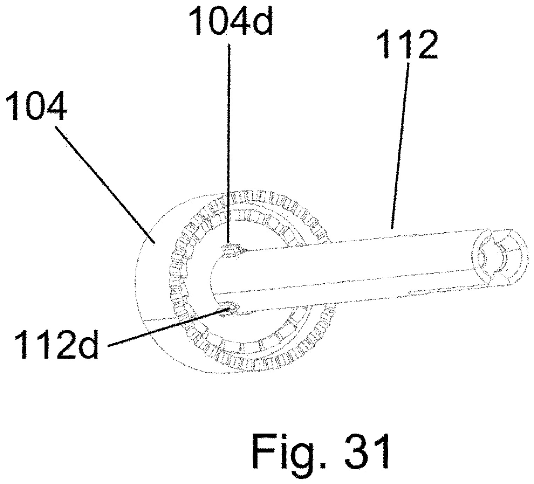

Fig. 31 shows the mutual arrangement of the driving sleeve 112 and the crown gear 104, which are rotationally coupled by means of the protrusions 112d on the external surface of the driving sleeve 112 and the grooves 104d on the crown gear 104.

The pen injector further comprises a threaded insert 118, which is best shown in figs. 27 and 28a-28b. As may be seen in fig. 28b, the piston rod 114 extends through the insert 118 so that an internal thread 118a of the insert 118 cooperates with an external thread 114b of the piston rod 114.

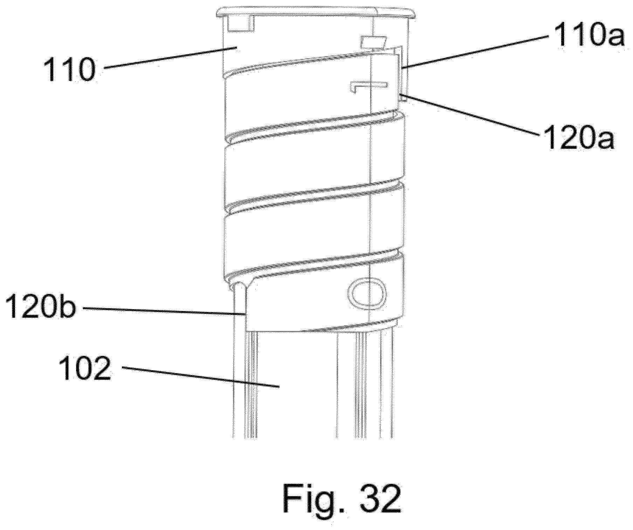

Figs. 32 show the arrangement of an indication member 120 and other parts of the injector. The indication member 120 is splined to the ratchet sleeve 102 and threadedly engaged with the housing 107 (not shown in Fig. 32). The currently set dose is visible on the indication member 120 through a window in the housing 107.

The fixed insert 110 is located within the knob 111 and it is attached inside the housing 107. As mentioned above, the drive spring 109 is attached to the fixed insert 110. The pen injector is equipped with a zero dose stop formed by the abutment of a vertical surface 120a of the indication member 120 and a corresponding vertical surface 110a of the fixed insert 110 as shown in fig. 32 showing the initial position of the indication member 120, being also the position where the indication member 120 is stopped after delivery of a dose. The indication member 120 comprises also a maximum dose stop 120b which limits the maximum dose which can be set by abutting a corresponding protrusion on the internal surface of the housing 107. A dose is set by rotation of the knob 111 and according to the operation of the dose setting-correcting mechanism of the first embodiment as it is described above. When the set dose is to be injected, the push button 113 is pressed causing the driving sleeve 112 to be pressed as well. In this way the driving sleeve protrusions 112c disengage the housing 107 and the mechanism becomes unlocked, while the driving sleeve 112 is still engaged with the crown gear 104. When the dose is set and the pen injector is ready for delivery, the drive spring 109 is tensioned, and when the mechanism is not blocked any more, the spring 109 rotates the ratchet sleeve 102 and its ratchet arm 102c rotate the setting ring 106 which in turn rotates the crown gear 104, which further rotates the driving sleeve 112 by means of their splined connection and the driving sleeve 112 rotates the piston rod 114 by means of the cooperating protrusions 112b and grooves 114a of these elements. In this way, the rotating piston rod 114 is also displaced axially in the distal direction due to its threaded coupling with the insert 118.

As may be seen in fig. 27, the pen injector according to the invention is also provided with a return spring 122 which is compressed during pressing the push button 113, because it is disposed between the driving sleeve 112 and the threaded insert 118, which does not move. After dose injection, the return spring 122 pushes the driving sleeve 112 back proximally to its initial position in which the driving sleeve 112 engages again the housing 107 and the mechanism is ready for the next injection.

### LIST OF ELEMENTS

Coupling sleeve 1, 101
   Proximal end 1a, 101a
   Distal end 1b, 101b
   Ratchet teeth 1c, 101c
   Protrusion(s) 1d, 101d
Ratchet sleeve 2, 102
   Proximal end 2a, 102a
   Distal end 2b, 102b
   Ratchet arm(s) 2c, 102c
   Groove(s) 2d, 102d
   Protrusion 102e
Reset nut 3, 103
   3a, 103a Ratchet arms
   3b, 103b reset teeth ring
Crown gear 4, 104
   tubular body 4a, 104a
   internal sliding crown teeth ring 4b, 104b
   external teeth ring 4c, 104c
      first set of slanted teeth 4e
      second set of slanted teeth 4f
   external longitudinal grooves 4d
   104d
Spring 5, 105
Setting ring 6, 106
   Proximal setting ring 6.1
      Coupling teeth ring 6.1a, 106a
      Ratchet teeth ring 6.1b, 106b
      Notches 6.1c
   Distal setting ring 6.2
      Stopping teeth ring 6.2a
      Projections 6.2b
Housing 7, 107
   Internal thread 7a, 107a
Cartridge holder 8, 108
Drive spring 9, 109
   Proximal end 9a, 109a
   Distal end 109b
Fixed insert 10, 110
Knob 11, 111
Driving sleeve 12, 112
   Proximal end 12a, 112a
   Flattened surface 12b
   Protrusion 112b
   Distal end 12c, 112c
   End of thread 12d
   Protrusions on the external surface 112d
Push button 13, 113
Piston rod 14, 114
   Flattened surface 14a
   Longitudinal groove 114a
   External thread 14b
Piston rod foot 15, 115
Nut 16
   Internal splines 16a
   External splines 16b
   Longitudinal projections 16c
Fixing ring 17
Threaded insert 18, 118
Bearing 19
Indication member 20, 120
   Vertical surface 20a, 120a
   Maximum dose stop 20b, 120b
Blocking ring 21
   Protrusion on internal surface
Return spring 22, 122

## Claims

1. A dose setting-correcting mechanism for a pen injector, comprising the following components arranged around a longitudinal axis (A):
a rotatable coupling sleeve (1, 101) having a proximal end (1a, 101a) and a distal end (1b, 101b),
a rotatable ratchet sleeve (2, 102) surrounding the coupling sleeve (1, 101) and splined therewith axially,
a reset nut (3, 103),
a setting ring (6, 106),
a crown gear (4, 104),
wherein the mechanism is configured for setting a dose by rotation of the coupling sleeve (1, 101) in a first direction which causes rotation of the ratchet sleeve (2, 102),
wherein
- the coupling sleeve (1, 101) is provided at its distal end (1b, 101b) with one element of a first pair comprising at least one ratchet arm and the ratchet teeth projecting transversally to the axis, and
- the reset nut (3, 103) is provided with the other of the said first pair so that a unidirectional rotational coupling is provided between the coupling sleeve (1, 101) and the reset nut (3, 103),
wherein
- the ratchet sleeve (2, 102) is provided at its distal end (2b, 102b) with one element of a second pair comprising at least one ratchet arm and the ratchet teeth projecting transversally to the axis, and
- the setting ring (6, 106) is provided with the other of the said second pair, so that a unidirectional rotational coupling is provided between the ratchet sleeve (2, 102) and the setting ring (6, 106),
wherein during dose setting the coupling sleeve (1, 101) rotates with respect to the reset nut (3, 103) and the ratchet sleeve (2, 102) rotates with respect to the setting ring (6, 106),
wherein the reset nut (3, 103) and the crown gear (4, 104) are coupled by first cooperating sets of axially extending teeth (3b, 103b, 4b, 104b) so that a unidirectional rotational coupling is provided between the reset nut (3, 103) and the crown gear (4, 104),
wherein the crown gear (4, 104) and the setting ring (6, 106) are releasably coupled by second cooperating sets of the axially extending teeth (4e, 104c, 6.1a, 106a),
wherein during dose correcting the coupling sleeve (1, 101) is firstly rotated with respect to the ratchet sleeve (2, 102) and the reset nut (3, 103) is rotated along with the coupling sleeve (1, 101), and
rotation of the reset nut (3, 103) causes sliding movement between the teeth (3b, 103b, 4b, 104b) and in result the axial movement of the crown gear (4, 104) and decoupling the crown gear (4, 104) from the setting ring (6, 106) so that the setting ring (6, 106) rotates to correct the dose.

2. The mechanism according to claim 1, wherein the setting ring (6) comprises a proximal setting ring (6.1) and a distal setting ring (6.2) fixed to each other.

3. The mechanism according to any of claim 1, wherein the teeth (103b, 104b) for coupling the crown gear (104) and the reset nut (103) comprise inclined surfaces, which are curved.

4. The mechanism according to any of the preceding claim, wherein the crown gear (4) further comprises a set of teeth (4f) and the setting ring (6) further comprises a set of teeth (6.2a), the sets of teeth (4f, 6.2a) being designed for stopping rotation during dose correcting and their respective teeth being spaced apart from each other along the longitudinal axis during dose setting and coupled with each other during dose correcting.

5. The mechanism according to claim 5, wherein the bases of the teeth (4f) are spaced from each other circumferentially and the bases of the teeth (6.2a) are spaced circumferentially.

6. The mechanism according to claim 1, wherein the teeth (104c) for coupling the crown gear (104) with the setting ring (106) are symmetrical.

7. The mechanism according to any preceding claim comprising a spring (5, 105) biasing the crown gear (4, 104)

8. The mechanism according to claim 7, wherein the spring (5, 105) is a helical compression spring.

9. A pen injector comprising the dose setting-correcting mechanism according to any of the preceding claims and further comprising:
a housing (7, 107) adapted to be coupled with a holder (8, 108) for medicament cartridge
a driving sleeve (12, 112) coupled to a push button (13, 113) at its proximal end (12a, 112a),
an externally threaded piston rod (14, 114),
wherein the driving sleeve (12, 112) is coupled in rotation with the crown gear (4, 104),
the driving sleeve (12, 112) is releasably coupled with the housing (7, 107), so that the driving sleeve (12, 112) is rotationally fixed during both dose setting and dose correcting and is released for injecting the dose,
wherein rotation of the driving sleeve (12, 112) causes distal displacement of the piston rod (14).

10. The pen injector according to claim 9, further comprising a drive spring (9, 109) for providing the force for injection.

11. The pen injector according to claim 10, wherein the drive spring (9, 109) is a helical torsion spring.

12. The pen injector according to any of claims 10-12, wherein the drive spring (9, 109) is coupled with the ratchet sleeve (2, 102).

13. The pen injector according to any of claims 9-12 wherein the driving sleeve (112) is releasably directly coupled to the housing (107).

14. The pen injector according to any of claims 9-13 comprising a proximally located button (13, 113) for initiating the injection.

15. The pen injector according to any of claims 9-14, wherein the pen injector further comprises a last dose stop mechanism preventing setting a dose higher than the remaining amount of drug in the cartridge.
